# NEUE EUROPÄISCHE PATENTSCHRIFT

(11) **EP 1 233 775 B2**
(45) Veröffentlichungstag und Bekanntmachung des Hinweises auf die Entscheidung über den Einspruch: **25.10.2006**
(45) Hinweis auf die Patenterteilung: 21.05.2003
(21) Anmeldenummer: 00987344.9
(22) Anmeldetag: 04.12.2000
(51) Int. Cl.: A61K 38/04, A61K 38/09, A61P 39/06, A61Q 17/00

(54) **TYROSIN- UND TRYPTOPHANHALTIGE PEPTIDE ALS ANTIOXIDANTIEN**
TYROSINE- AND TRYPTOPHAN-CONTAINING PEPTIDES AS ANTIOXIDANTS
PEPTIDES RENFERMANT DE LA TYROSINE ET DU TRYPTOPHANE UTILISES COMME ANTIOXYDANTS

(30) Priorität: 02.12.1999 DE 19958121
(43) Veröffentlichungstag der Anmeldung: 28.08.2002
(73) Patentinhaber: MAX-PLANCK-GESELLSCHAFT ZUR FÖRDERUNG DER WISSENSCHAFTEN E.V., 80539 München (DE)
(72) Erfinder: MOOSMANN, Bernd, 81547 München (DE); BEHL, Christian, 80638 München (DE)
(74) Vertreter: Weiss, Wolfgang
(86) Internationale Anmeldenummer: PCT/EP2000/012177
(87) Internationale Veröffentlichungsnummer: WO 2001/039791

(56) Entgegenhaltungen:
- EP-A- 0 173 181
- EP-B- 0 871 611
- WO-A-91/12267
- WO-A-99/02163
- DE-A- 2 932 923
- DE-A- 3 836 849
- GB-A- 536 406
- US-A- 5 736 509
- US-A- 5 807 830
- MOOSMANN BERND ET AL: "Cytoprotective antioxidant function of tyrosine and tryptophan residues in transmembrane proteins." EUROPEAN JOURNAL OF BIOCHEMISTRY, Bd. 267, Nr. 18, September 2000 (2000-09), Seiten 5687-5692, XP002173184 ISSN: 0014-2956
- Prasad, G. et al.; J. Neurochemistry, 73(4):1660, 1999
- Camoretti-Mercado,B. et al.; Eur. J. Biochem., 156:317-325, 1986
- Klinguer, C. et al.; Vaccine, 18:259-267, 1999
- JP-A-06/345797 (Derwent Abstract)
- Abstract von Adv. Exp. Med. Biol., 1999, 467:541-52
- Chen et al.; J. Agric. Food Chem., 1996, 44, pp. 2619-2623
- Chen et al.; J. Agric. Food Chem., 1995, 43, pp. 574-578

## Beschreibung

Die Erfindung betrifft die Verwendung von tryptophanhaltigen, insbesondere tryptophan- und tyrosinhaltigen Peptiden als Antioxidantien. Die Verbindungen können zur Therapie oder Prophylaxe von Syndromen oder Erkrankungen dienen, die mit unerwünschten oxidativen Prozessen im Extrazellulärraum einhergehen.

Viele Erkrankungen stehen mit einer erhöhten oxidativen Belastung in Zusammenhang. Diese kann zu unterschiedlichen zellulären Reaktionen wie etwa Differenzierung, Apoptose, Nekrose oder oxidative Zellyse führen (Irani et al., Science 275 (1997), 1649; Burdon, Free Radic. Biol. Med. 18 (1995), 775; Anderson et al., Med. Hypotheses 52 (1999), 451; Hampton und Orrenius, Toxicol. Lett. 102-103 (1998), 355; Kowaltowski und Vercesi, Free Radic. Biol. Med. 26 (1999), 463; Beal, Ann. Neurol. 38 (1995), 357).

Auch der Extrazellulärraum kann durch oxidative Prozesse in seiner Struktur und Funktionalität beeinflußt werden, was zu nachteiligen biochemischen und physiologischen Veränderungen führen kann. Beispiele sind die Oxidation von Low Density Lipoprotein (LDL), die ursächlich an artherosklerotischen Prozessen beteiligt ist (Heinecke, J. Clin. Invest. 104 (1999), 135; Parthasarathy, Annu. Rev. Med. 43 (1992), 219), die zu Katarakten führenden Veränderungen im Aggregationsverhalten von Linsenproteinen (Christen, Proc. Assoc. Am. Physicians 111 (1999), 16; Taylor und Nowell, Adv. Pharmacol. 3B (1997), 515) oder oxidative Quervemetzungen von Kollagen, die bei Diabetes, Arthritis oder Alterungsprozessen gefunden werden (Bailey et al., Mech. Ageing Dev. 106 (1998), 1; Beckmann und Ames, Physiol. Rev. 78 (1998), 547). Hinzu kommt, dass die Anzahl bekannter Antioxidationssysteme im Extrazellulärraum geringer als innerhalb der Zelle ist (van der Vlies et al., Am. J. Physiol. 276 (1999), L289; Frei et al., Proc. Natl. Acad. Sci. USA 85 (1988), 9748; Mimic-Oka et al., Clin. Nephrol. 51 (1999), 233; Cross et al., Environ. Health Perspect. 106 Suppl 5 (1998), 1241; Wilson, Can. J. Physiol. Pharmacol. 75 (1997),1149; Grootveld et al., Fee Radic. Res. 30 (1999), 351; Sies, Exp. Physiol. 82 (1997), 291).

Es besteht daher ein großes Bedürfnis nach Substanzen, die einerseits als Antioxidationsmittel im Extrazellulärraum eine hohe Wirkung aufweisen und andererseits physiologisch verträglich sind.

In den zur vorliegenden Erfindung vorliegenden Untersuchungen wurde eine neue Klasse biochemischer Antioxidationsmittel des Extrazellulärraums identifiziert. Dabei handelt es sich um niedermolekulare lösliche Peptide, die aromatische Ringsysteme, mit der Fähigkeit zur Ausbildung stabiler Radikale, insbesondere Tryptophanreste, bevorzugt Tryptophan- und Tyrosinreste enthalten. Diese Peptide verhindern bzw. verringern die Oxidation von physiologisch bedeutsamen Komponenten des extrazellulären Raums, z.B. gelösten Proteinen, fibrillären Proteinen, Lipoproteinpartikeln und Membranlipiden. Da eine Oxidation dieser biologischen Komponenten bei einer Vielzahl von degenerativen Erkrankungen auftritt, stellt die Erfindung eine wichtige neue Erkenntnis auf dem Gebiet der Medizin dar.

Die erfindungsgemäßen Antioxidationsmittel sind chemisch einfach und preiswert in großen Mengen herzustellen, während bekannte extrazellulär wirksame Antioxidantien (z.B. Ascorbat, Tocopherol, Ubichinol) entweder erheblich teurer (Tocopherol, Ubichinol) oder weniger wirksam sind (Ascorbat). Die erfindungsgemäßen Verbindungen sind auch wesentlich beständiger als die bekannten Antioxidantien, da sie gegenüber Luftsauerstoff im wesentlichen stabil sind, aber gegenüber gewebsschädigenden, auf Sauerstoff basierenden Radikalen eine hohe Reaktivität zeigen. Weiterhin sind die Verbindungen amphiphil, d.h. sie sind wasserlöslich, lassen sich aber auch z.B. durch Ultraschallbehandlung in lipophile Formulierungen z.B. Liposomen auf technisch einfache Weise einbauen. Noch ein weiterer Vorteil ist, dass die erfindungsgemäßen Verbindungen intakte Zellen nicht penetrieren, d.h. sie lassen sich bereits bei geringen Dosierungen im Extrazellulärraum anreichern. Außerdem können natürliche, vollständig aus endogen vorkommenen Aminosäuren bestehende Verbindungen eingesetzt werden, die keine metabolisch akkumulativen oder toxischen Effekte aufweisen. Schließlich zeigen die Verbindungen nur geringe Neigung zur Plasmaproteinbindung, so dass die frei diffusiblen, wirksamen Konzentrationen höher sind.

Ein Gegenstand der Erfindung ist somit die Verwendung von Verbindungen, die (a) mindestens einen Tryptophanrest und mindestens einen Tyrosinrest oder mindestens zwei Tryptophanreste enthalten oder/und analoge aromatische Reste oder/und (b) mindestens zwei Tyrosinreste oder/und analoge aromatische Reste enthalten und eine Molekularmasse von bis zu 2.500 Da aufweisen, zur Herstellung eines Mittels für die Hemmung oxidativer Prozesse im Extrazellulärraum. Insbesondere eignen sich die Verbindungen zur Therapie oder Prophylaxe von Erkrankungen, die mit oxidativen Prozessen im Extrazellulärraum einhergehen, beispielsweise von degenerativen Erkrankungen, wie etwa Artherosklerose, Diabetes, Katarakten, Arthritis und Alterungsprozessen in Gelenken oder Haut. Besonders bevorzugt werden die Verbindungen in Hautcremes zur Verlangsamung der Hautalterung, insbesondere vor, während, oder nach UV-Exposition sowie als Prophylaktikum oder Medikament bei arthritischen oder rheumatischen oxidativen Prozessen sowie bei Artherosklerose eingesetzt.

Die Verbindungen sind in der Lage, den Beginn oder das Fortschreiten oxidativer Prozesse an physiologischen Komponenten des Extrazellulärraumes wie etwa löslichen Proteinen, fibrillären Proteinen, z.B. Kollagen, Glykoproteinen, Proteoglykanen, Lipoproteinen, z.B. Lipoproteinpartikeln wie LDL, und Membranlipiden zu hemmen. Vorzugsweise werden niedermolekulare Peptide mit amphiphilen Eigenschaften eingesetzt, d.h. Verbindungen, die einerseits wasserlöslich sind, andererseits eine Affinität für unpolare organische Reste aufweisen. In einer Ausführungsform enthalten die Peptide mindestens einen Tryptophanrest oder einen analogen aromatischen Rest und zusätzlich mindestens einen Tyrosinrest oder einen analogen aromatischen Rest. In einer weiteren Ausführungsform enthalten die Peptide mindestens zwei Tyrosinreste oder analoge aromatische Reste und gegebenenfalls zusätzlich mindestens einen Tryptophanrest oder einen analogen aromatischen Rest. Beispiele für geeignete analoge Reste sind insbesondere aromatische Aminosäurereste mit einem Phenol- oder einem Tryptophan-analogen Indol-Ringsystem, wie etwa einen Alkyloxy-Phenol- oder Alkyloxy-Indol-Ring, insbesondere einen Methoxy-Phenol- oder Methoxy-Indol-Ring. Die antioxidativ wirksamen Aminosäurereste können in der L- oder/und in der D-Konformation vorliegen. Bevorzugt ist die L-Konformation.

In einer Ausführungsform der Erfindung werden die Verbindungen aus Peptiden mit einer Länge von 2 bis 15, vorzugsweise 2 bis 10 und besonders bevorzugt 2 bis 7 Aminosäuren ausgewählt. Peptide im Sinne der vorliegenden Erfindungen sind im wesentlichen aus Aminosäuren, insbesondere aus α-Aminocarbonsäuren, z.B. aus natürlichen genetisch kodierten Aminosäuren aber auch aus anderen Aminosäuren, aufgebaute Verbindungen, wobei die Aminosäurereste über Peptidbindungen miteinander verknüpft sind. Weiterhin können die Peptide gegebenenfalls N- oder/und C-terminale Modifikationen, z.B. eine N-terminale Acylierung oder eine C-terminale Veresterung oder Amidierung oder eine cyclische Struktur aufweisen.

Die Verbindungen enthalten mindestens zwei Tyrosin-, Tryptophan- oder/und analoge aromatische Reste, z.B. mindestens zwei Tyrosinreste, mindestens einen Tryptophan-und einen Tyrosinrest oder mindestens zwei Tryptophanreste. Dabei hat sich gezeigt, dass eine räumliche Nachbarschaft dieser Reste wünschenswert sein kann, z.B. im Falle von peptidischen Verbindungen sind die antioxidativ wirkenden Reste vorzugsweise entweder direkt benachbart bzw. durch eine oder maximal zwei dazwischenliegende Aminosäurereste getrennt. Weiterhin ist bevorzugt, dass die Verbindungen bis zu vier der antioxidativ wirksamen Reste enthalten. Es gibt jedoch Fälle, in denen die Verbindungen auch eine höhere Anzahl an antioxidativ wirksamen Resten enthalten können.

Die zur vorliegenden Erfindungen führenden Untersuchungen wurden an physiologisch wirksamen Peptidhormonen mitTryptophanresten, bevorzugt Tryptophan- und Tyrosinresten, durchgeführt. Dabei wurde gefunden, dass die antioxidative Wirkung nicht mit anderen strukturspezifischen physiologischen Wirkungen dieser Peptidhormone, sondern lediglich mit dem Vorhandensein von Tryptophanresten, insbesondere Tryptophan- und Tyrosinresten, in Korrelation steht. Um unerwünschte physiologische Nebenwirkungen im Rahmen einer medizinischen Behandlung mit den erfindungsgemäßen Verbindungen möglichst weitgehend zu vermeiden, werden daher die erfindungsgemäßen Verbindungen, z.B. Peptide so ausgewählt, dass sie im wesentlichen keine strukturspezffische physiologische Wirkung, z.B. eine Hormonwirkung aufweisen.

Beispiele für antioxidativ wirksame sekretorische Peptide sind in der folgenden Tabelle aufgelistet.

**Tabelle**

| **Peptid** | **Aminosäuresequenz** |
|---|---|
| GnRH (*gonadotrophin-releasing hormone*) (Mensch) | cyEHWSYGLRPG-amid |
| GnRH (*gonadotrophin-releasing hormone)* (Lachs) | cyEHWSYGWLPG-amid |
| GnRH (*gonadotrophin-releasing hormone)* (Neunauge) | cyEHWSHDWKPG-amid |
| Dermorphin | Y^{D}-AFGY PS-amid |
| cyE = cyclisches Glutamat | |
| X^{D} = D-Aminosäure | |

Neben diesen Peptiden sind auch alle trunkierten Fragmente davon wirksam, soweit deren Tyrosin- oder Tryptophan-Gehalt nicht verringert ist.

Beispiele von synthetischen und als Antioxidationsmittel wirksamen Peptiden, die jedoch keine Hormonwirkung besitzen, sind WY, WGY, WGGY, WGGGY, WW und YY.

Die Verabreichung der Verbindungen erfolgt in Abhängigkeit von der Art bzw. Schwere der zu behandelnden Erkrankung sowie von der Art der Verabreichung. Vorzugsweise erfolgt die Verabreichung so, dass am Zielort eine Wirkstoffkonzentration im Bereich von 1 nM bis 100 µM, insbesondere 2 nM bis 20 µM vorliegt. Die Verabreichung kann systemisch, z.B. durch intravaskuläre Injektion, insbesondere jedoch durch orale Gabe erfolgen. Andererseits kann die Verabreichung jedoch auch lokal, z.B. durch intramuskuläre oder subkutane Injektion, insbesondere jedoch durch topische Applikation erfolgen.

In einer besonders bevorzugten Ausführungsform der Erfindung werden die antioxidativ wirksamen Substanzen einer Sonnencreme-Formulierung zugesetzt, um Schädigungen der Haut durch UV-Exposition zu vermeiden. Ein Gegenstand der Erfindung ist somit auch eine Sonnencreme-Formulierung, die neben üblichen Bestandteilen einen antioxidativ wirksamen Anteil von Peptiden mit mindestens einem Tryptophanrest und mindestens einem Tyrosinrest oder mindestens zwei Tryptophanresten oder/und analogen aromatischen Resten oder/und mindestens zwei Tyrosinresten oder/und analogen aromatischen Resten und einer Molekularmasse von bis zu 2500 Da enthält. Der Anteil der antioxidativ wirksamen Peptide in der Sonnencreme-Formulierung ist vorzugsweise von 0,5 bis 10 Gew.%.

Weiterhin soll die Erfindung durch die nachfolgenden Figuren und Beispiele erläutert werden. Es zeigen:
- Figur 1: die antioxidative Wirkung von tryptophanhaltigen, insbesondere tryptophan- und tyrosinhaltigen Peptiden beim Abfang von Peroxyradikalen (A), bei der Verhinderung der LDL-Oxidation (B) und bei der Verhinderung der Peroxidation von Gehirnlipiden (C), und .
- Figur 2: die essentielle Bedeutung von Tryptophanresten, insbesondere Tryptophan- und Tyrosinresten für die antioxidative Wirkung von Peptiden, und
- Figur 3: die antioxidative Wirkung von Vergleichssubstanzen.

### Beispiele

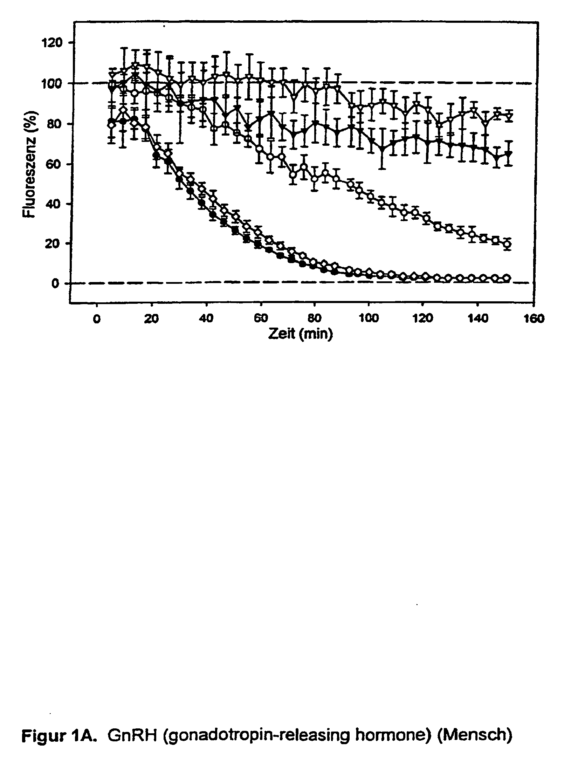

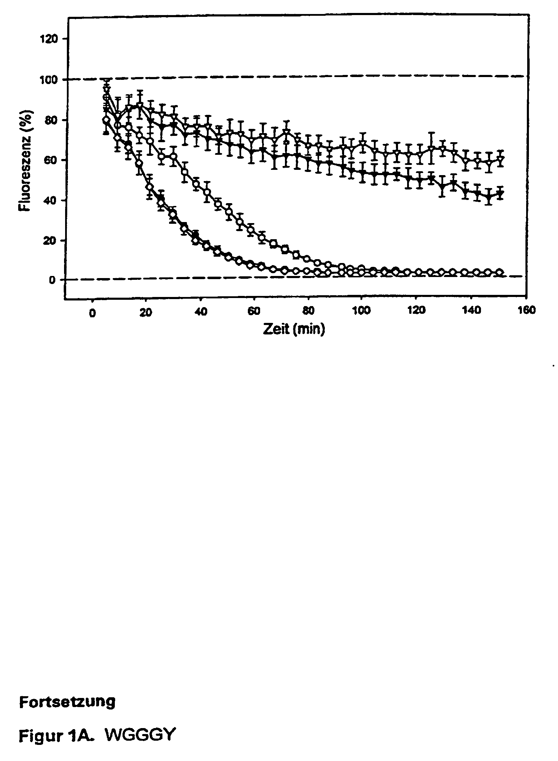

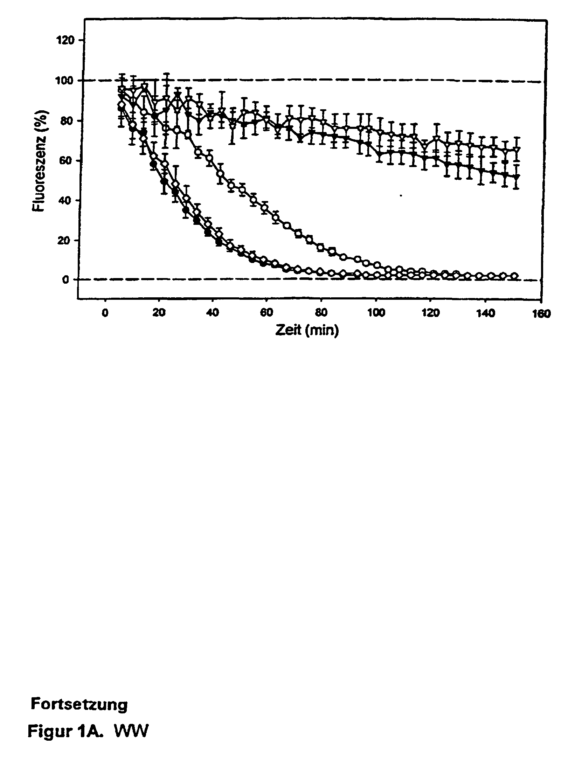

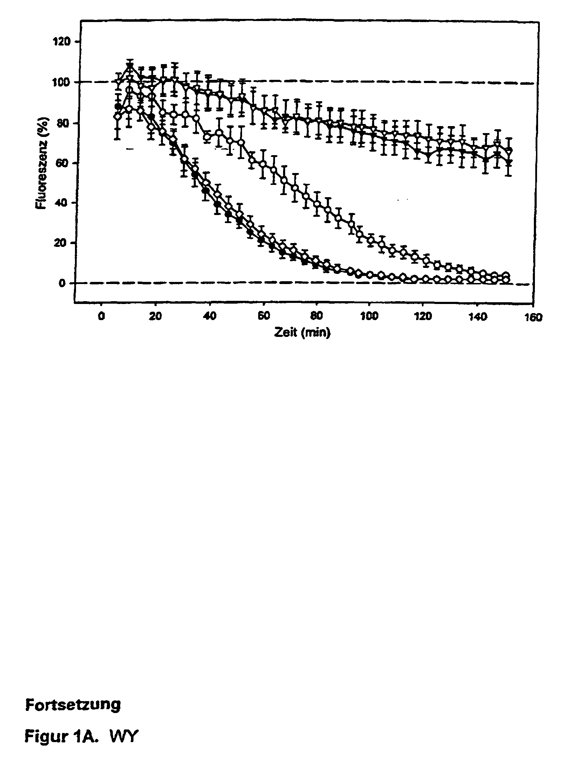

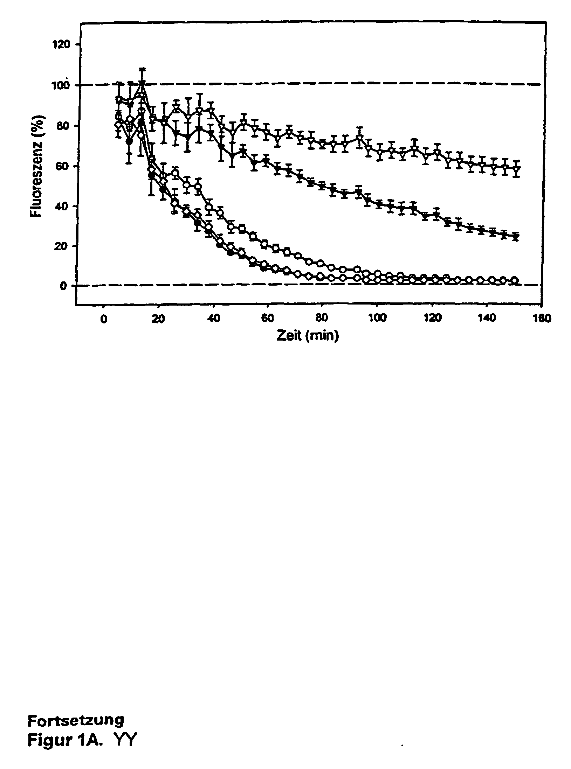

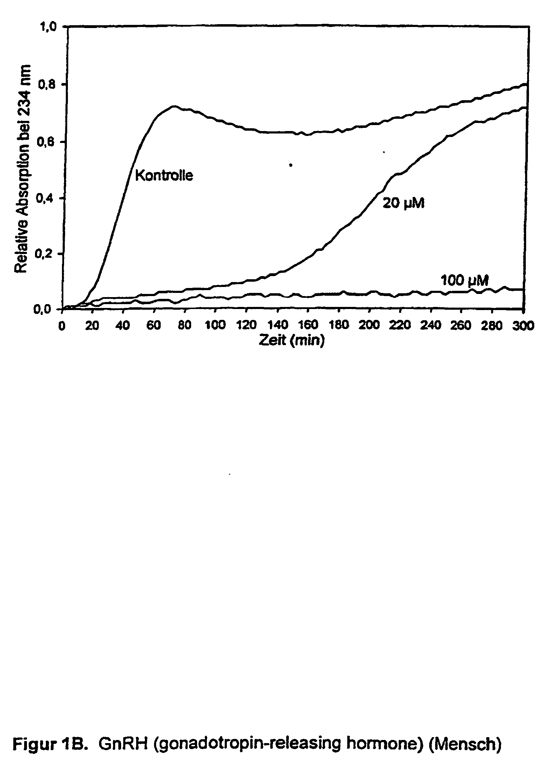

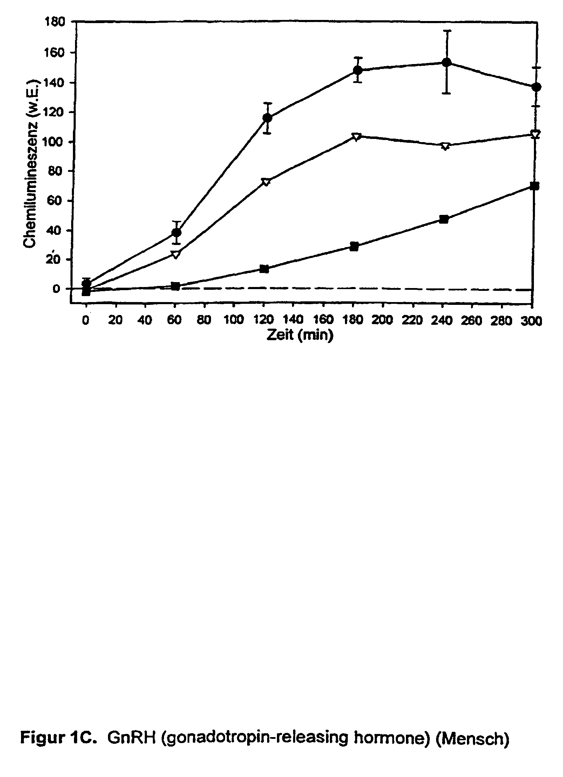

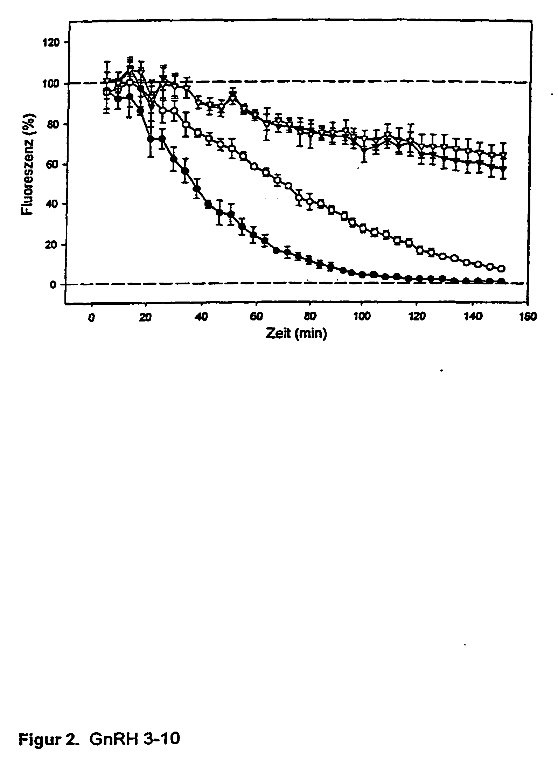

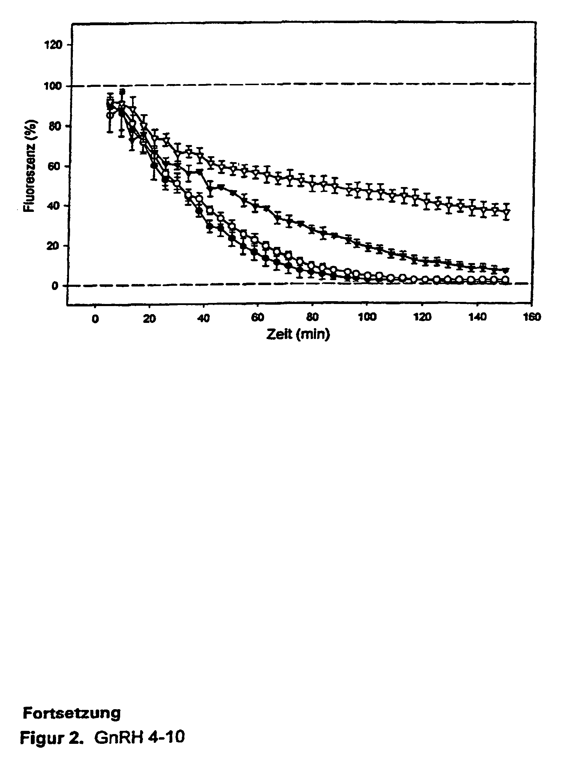

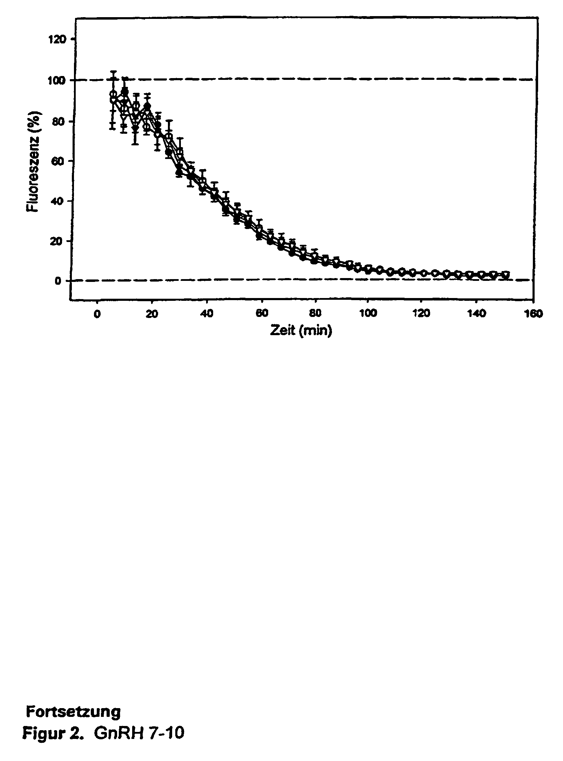

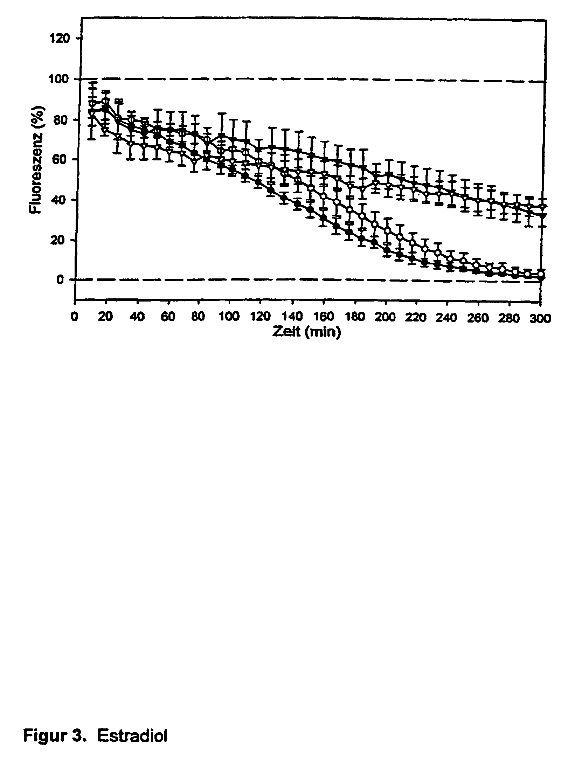

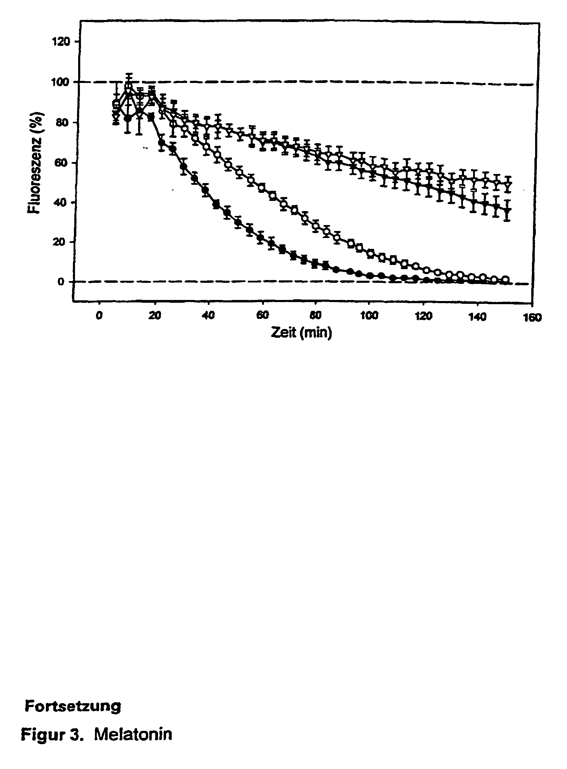

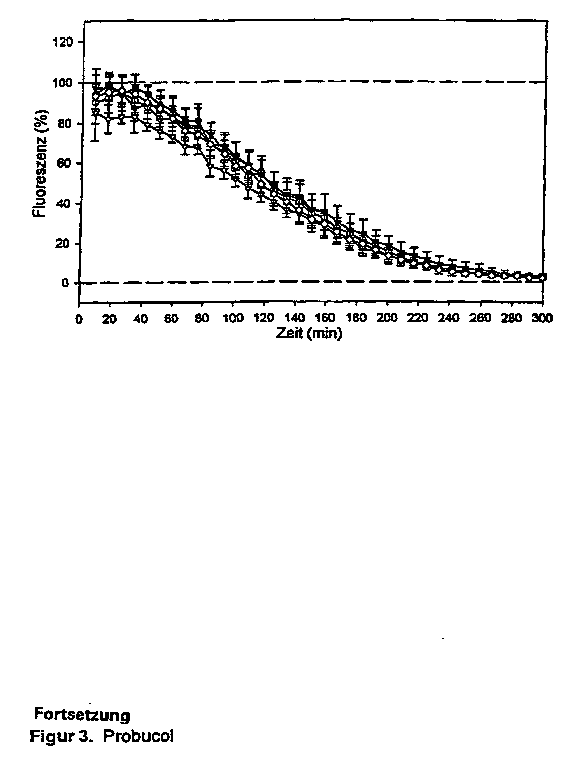

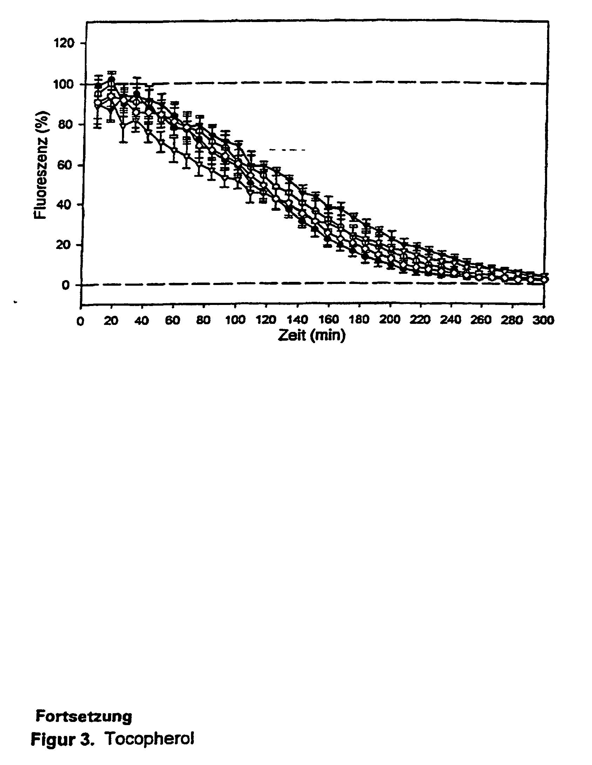

1.
   Es wurde die Fähigkeit von Peptiden, das globuläre Protein B-Phycoerythrin vor einer Zerstörung durch Peroxyradikale zu schützen, getestet. Hierzu wurde eine Oxidation von B-Phycoerythrin von Porphyrium cruentum (10 nM) in Phosphatgepufferte Salzlösung unter Verwendung von 2,2'-Azo-bis-2-methylpropionamidin (500 µM) bei 37°C als Quelle freier Peroxyradikale durchgeführt. Die Zerstörung des Proteins wurde durch Beobachtung der Abnahme der Proteinfluoreszenz bei steigender Inkubationsdauer gemessen (Flash-Fluorimetrie, Anregungsfenster 340±50 nm, Emissionsfenster 572±6 nm). Die Ergebnisse dieses Versuchs für das sekretorische Peptide GnRH (Mensch) sowie für die synthetischen Peptide WY, WGGGY, WW und YY sind in Figur 1A dargestellt. In den einzelnen Abbildungen bedeutet ● die Kontrolle und ◊ eine Konzentration von 20 nM, o 200 nM, ▼2 µM bzw. ∇20 µM des jeweiligen Peptids (Vierfachbestimmungen). Bei den sekretorischen Peptiden GnRH (Lachs), GnRH (Neunauge) und Dermorphin sowie für die synthetischen Peptide WGY und WGGY wurden analoge Ergebnisse erhalten.
   In einem weiteren Test wurde die Fähigkeit der Peptide zum Schutz von LDL vor einer Kupfer-katalysierten Oxidation getestet. Hierzu wurde frisches humanes LDL (0,1 mg/ml Protein) mit 10 µM CuSO₄ bei 37°C inkubiert. Die Reaktionsprodukte der LDL-Zersetzung (konjugierte Diene) wurden photometrisch bei 234 nm wie bei Moosmann und Behl (Proc. Natl. Acad. Sci. USA 96 (1999), 8867) beschrieben, gemessen. Die Ergebnisse für das sekretorische Peptid GnRH (Mensch) sind in Figur 1 B zu erkennen. Die drei Kurven zeigen die Kontrolle, eine Konzentration von 20 µM sowie eine Konzentration von 100 µM des jeweiligen Peptids (Doppelbestimmungen).
   Sekretorische Peptide können Gehirnmembranen vor einer oxidativen Zerstörung durch Ascorbat schützen. Hierzu wurde native Rattenhimmembran (0,5 mg/ml Protein) durch Zugabe von 50 µM Ascorbat bei 37°C oxidiert. Eine Einzelphotonenmessung der Chemilumineszenz (als Maß für auftretende Peroxidationsreaktionen) wurde zur quantitativen Bestimmung der Lipidperoxidation wie bei Moosmann und Behl (supra) beschrieben gemessen. Die Ergebnisse für GnRH (Mensch) sind in Figur 1C gezeigt. ● bedeutet die Kontrolle und 0 eine Konzentration von 20 µM und ■ eine Konzentration von 100 µM des jeweiligen Peptids (Doppelbestimmungen).
   Aus Figur 1 ist ersichtlich, dass kurze, lösliche, tryptophanhaltige, insbesondere tryptophan- und tyrosinhaltige sekretorische Peptide wie Gonadotrophin-freisetzendes Hormon (GnRH) sowie synthetische Peptide antioxidative Eigenschaften aufweisen.
2.
   Um nachzuweisen, dass Tryptophan, insbesondere Tryptophan und Tyrosin, die Antioxidationswirkung der sekretorischen Peptide vermitteln, wurde das Verhalten von modifizierten odertrunkierten Peptiden im Peroxyradikal-Abfangtest gemäß Beispiel 1 untersucht. Die Ergebnisse sind in Figur 2 gezeigt. ● bedeutet die Kontrolle, ○bedeutet eine Konzentration von 200 nM, ▼ bedeutet eine Konzentration von 2 µM und ∇ eine Konzentration von 20 µM des jeweiligen Peptids (Vierfachbestimmungen).
   Figur 2 zeigt auch die antioxidative Wirkung von trunkierten GnRH-Fragmenten. Während bei GnRH 3-10 (WSYGLRPG-amid) eine ähnliche Wirkung wie bei GnRH (cy-EHWSYGLRPG-amid) auftritt, wird die Antioxidationswirkung bei GnRH 4-10 (SYGLRPG-amid), dem der Tryptophanrest fehlt, deutlich verringert. Eine weitere Verkürzung verbunden mit einem Fehlen des Tyrosinrests wie bei GnRH 7-10 (LRPG-amid) führt zu einem vollständigen Aktivitätsverlust.
3.
   Es wurden Versuche durchgeführt, in denen die antioxidative Wirkung anderer Substanzen im Peroxyradikal-Abfangtest gemäß Beispiel 1 getestet wurde. Die Ergebnisse sind in Figur 3 gezeigt (Erläuterung siehe Figur 1 A). Dabei wurde gefunden, dass die Peptide eine höhere antioxidative Wirkung als bekannte Antioxidationsmittel wie Tocopherol und Probucol und eine mindestens vergleichbare Wirkung wie Melatonin und 17β-Estradiol haben.

## Patentansprüche

1. Verwendung von Peptiden, die (a) mindestens einen Tryptophanrest und mindestens einen Tyrosinrest oder mindestens zwei Tryptophanreste enthalten oder/und analoge aromatische Reste oder/und (b) mindestens zwei Tyrosinreste oder/und analoge aromatische Reste enthalten und eine Molekularmasse von bis zu 2500 Da aufweisen, zur Herstellung eines Mittels für die Hemmung oxidativer Prozesse im Extrazellulärraum.

2. Verwendung nach Anspruch 1 zur Therapie oder Prophylaxe von Erkrankungen, die mit oxidativen Prozessen im Extrazellulärraum einhergehen.

3. Verwendung nach Anspruch 2 zur Therapie oder Prophylaxe von degenerativen Erkrankungen.

4. Verwendung nach Anspruch 2 oder 3 zur Therapie oder Prophylaxe von Erkrankungen ausgewählt aus Artherosklerose, Diabetes, Katarakten, Arthritis und Alterungsprozessen in Gelenken oder in Haut.

5. Verwendung nach einem der vorhergehenden Ansprüche,
**dadurch gekennzeichnet,**
**dass** oxidative Prozesse an physiologischen Komponenten des Extrazellulärraumes gehemmt werden.

6. Verwendung nach Anspruch 5,
**dadurch gekennzeichnet,**
**dass** die physiologischen Komponenten ausgewählt werden aus löslichen Proteinen, fibrillären Proteinen, Lipoproteinen, Glykoproteinen, Proteoglykanen und Membranlipiden.

7. Verwendung nach einem der vorhergehenden Ansprüche,
**dadurch gekennzeichnet,**
**dass** die Peptide amphiphile Eigenschaften haben.

8. Verwendung nach einem der vorhergehenden Ansprüche,
**dadurch gekennzeichnet,**
**dass** die Peptide eine Länge von 2 bis 15 Aminosäuren besitzen und gegebenenfalls N- oder/und C-terminale Modifikationen oder eine cyclische Struktur aufweisen.

9. Verwendung nach Anspruch 8,
**dadurch gekennzeichnet,**
**dass** die Peptide eine Länge von 2 bis 10 Aminosäuren aufweisen.

10. Verwendung nach einem der Ansprüche 1 bis 9,
**dadurch gekennzeichnet,**
**dass** die Peptide eine Länge von 2 bis 7 Aminosäuren aufweisen.

11. Verwendung nach einem der vorhergehenden Ansprüche,
**dadurch gekennzeichnet,**
**dass** die Verbindungen mindestens zwei Tyrosin-, Tryptophan- oder/und analoge aromatische Reste enthalten.

12. Verwendung nach Anspruch 11,
**dadurch gekennzeichnet,**
**dass** die Verbindungen bis zu vier Tyrosin-, Tryptophan- oder/und analoge aromatische Reste enthalten.

13. Verwendung nach einem der Ansprüche 8 bis 12,
**dadurch gekennzeichnet,**
**dass** die Peptide so ausgewählt werden, dass sie im wesentlichen keine strukturspezifische physiologische Wirkung aufweisen.

14. Verwendung nach einem der Ansprüche 8 bis 13,
**dadurch gekennzeichnet,**
**dass** die Peptide ausgewählt werden aus Gonadotrophin-freisetzendem Hormon, Dermorphin sowie N- oder/und C-terminal trunkierten Fragmenten davon.

15. Verwendung nach einem der Ansprüche 8 bis 13,
**dadurch gekennzeichnet,**
**dass** die Peptide ausgewählt werden aus der Gruppe bestehend aus WY, WGY, WGGY, WGGGY, WW und YY.

16. Verwendung nach einem der vorhergehenden Ansprüche,
**dadurch gekennzeichnet,**
**dass** eine Verabreichung der Verbindungen derart erfolgt, dass am Zielort eine Wirkstoffkonzentration im Bereich von 1 nM bis 100 µM vorliegt.

17. Verwendung nach einem der vorhergehenden Ansprüche,
**dadurch gekennzeichnet,**
**dass** die Verabreichung systemisch, insbesondere durch orale Gabe erfolgt.

18. Verwendung nach einem der vorhergehenden Ansprüche,
**dadurch gekennzeichnet,**
**dass** die Verabreichung lokal, insbesondere durch topische Applikation erfolgt.

19. Sonnencreme-Formulierung,
**dadurch gekennzeichnet,**
**dass** sie neben üblichen Bestandteilen einen antioxidativ wirksamen Anteil von Peptiden mit (a) mindestens einem Tryptophanrest und mindestens einem Tyrosinrest oder mindestens zwei Tryptophanresten oder/und analogen aromatischen Resten oder/und (b) mindestens zwei Tyrosinresten oder/und analogen aromatischen Resten und einer Molekularmasse von bis zu 2500 Da enthält.

20. Formulierung nach Anspruch 19,
**dadurch gekennzeichnet,**
**dass** die Peptide in einem Anteil von 0,5 bis 10 Gew.-% bezogen auf das Gesamtgewicht der Formulierung vorliegen.

## Claims

1. Use of peptides which (a) contain at least one tryptophan residue and at least one tyrosine residue or at least two tryptophan residues or/and analogous aromatic residues or/and (b) contain at least two tyrosine residues or/and analogous aromatic residues, and have a molecular mass of up to 2500 Da to produce an agent for inhibiting oxidative processes in the extracellular space.

2. Use as claimed in claim 1 for the treatment or prophylaxis of diseases that are associated with oxidative processes in the extracellular space.

3. Use as claimed in claim 2 for the treatment or prophylaxis of degenerative diseases.

4. Use as claimed in claim 2 or 3 for the treatment or prophylaxis of diseases selected from arteriosclerosis, diabetes, cataracts, arthritis and ageing processes in the joints or skin.

5. Use as claimed in one of the previous claims,
**characterized in that**
oxidative processes on physiological components of the extracellular space are inhibited.

6. Use as claimed in claim 5,
**characterized in that**
the physiological components are selected from soluble proteins, fibrillary proteins, lipoproteins, glycoproteins, proteoglycans and membrane lipids.

7. Use as claimed in one of the previous claims,
**characterized in that**
the peptides have amphiphilic properties.

8. Use as claimed in one of the previous claims,
**characterized in that**
the peptides have a length of 2 to 15 amino acids and optionally have N-terminal or/and C-terminal modifications or a cyclic structure.

9. Use as claimed in claim 8,
**characterized in that**
the peptides have a length of 2 to 10 amino acids.

10. Use as claimed in one of the claims 1 to 9,
**characterized in that**
the peptides have a length of 2 to 7 amino acids.

11. Use as claimed in one of the previous claims,
**characterized in that**
the compounds contain at least two tyrosine, tryptophan or/and analogous aromatic residues.

12. Use as claimed in claim 11,
**characterized in that**
the compounds contain up to four tyrosine, tryptophan or/and analogous aromatic residues.

13. Use as claimed in one of the claims 9 to 12,
**characterized in that**
the peptides are selected such that they have essentially no structure-specific physiological action.

14. Use as claimed in one of the claims 8 to 13,
**characterized in that**
the peptides are selected from gonadotrophin-releasing hormone, dermorphin and N-terminally or/and C-terminally truncated fragments thereof.

15. Use as claimed in one of the claims 8 to 13,
**characterized in that**
the peptides are selected from the group comprising WY, WGY, WGGY, WGGGY, WW and YY.

16. Use as claimed in one of the previous claims,
**characterized in that**
the compounds are administered in such a manner that a concentration of active substance in the range of 1 nM to 100 µM is present at the target site.

17. Use as claimed in one of the previous claims,
**characterized in that**
the administration takes place systemically and in particular by oral administration.

18. Use as claimed in one of the previous claims,
**characterized in that**
the administration takes place locally and in particular by topical application.

19. Sun cream formulation,
**characterized in that**
in addition to the usual components, it contains an anti-oxidatively effective amount of peptides containing (a) at least one tryptophan residue and at least one tyrosine residue or at least two tryptophan residues or/and analogous aromatic residues or/and (b) at least two tyrosine residues or/and analogous aromatic residues and has a molecular mass of up to 2500 Da.

20. Formulation as claimed in claim 19,
**characterized in that**
the peptides are present in an amount of 0.5 to 10 % by weight based on the total weight of the formulation.

## Revendications

1. Utilisation de peptides qui contiennent (a) au moins un radical tryptophane et au moins un radical tyrosine ou au moins deux radicaux tryptophane et/ou contiennent des radicaux aromatiques analogues et/ou (b) au moins deux radicaux tyrosine et/ou des radicaux aromatiques analogues et ont une masse moléculaire jusqu'à 2 500 Da, pour préparer un agent destiné à inhiber des processus oxydants dans l'espace extracellulaire.

2. Utilisation selon la revendication 1, pour traiter ou pour prévenir des maladies associées à des processus oxydants dans l'espace extracellulaire.

3. Utilisation selon la revendication 2, pour traiter ou pour prévenir des maladies dégénératives.

4. Utilisation selon la revendication 2 ou 3, pour traiter ou pour prévenir des maladies choisies parmi l'artériosclérose, le diabète, les cataractes, l'arthrite et des processus de vieillissement des articulations ou de la peau.

5. Utilisation selon l'une des revendications précédentes, **caractérisée en ce que** les processus oxydants sont inhibés sur des composants physiologiques de l'espace extracellulaire.

6. Utilisation selon la revendication 5, **caractérisée en ce que** les composants physiologiques sont choisis parmi les protéines solubles, les protéines fibrillaires, les lipoprotéines, les glycoprotéines, les protéoglycanes et les lipides membranaires.

7. Utilisation selon l'une des revendications précédentes, **caractérisée en ce que** les peptides ont des propriétés amphiphiles.

8. Utilisation selon l'une des revendications précédentes, **caractérisée en ce que** les peptides ont une longueur de 2 à 15 acides aminés et ont éventuellement des modifications N- ou C-terminales ou une structure cyclique.

9. Utilisation selon la revendication 8, **caractérisée en ce que** les peptides ont une longueur de 2 à 10 acides aminés.

10. Utilisation selon l'une des revendications 1 à 9, **caractérisée en ce que** les peptides ont une longueur de 2 à 7 acides aminés.

11. Utilisation selon l'une des revendications précédentes, **caractérisée en ce que** les composés contiennent au moins deux radicaux tyrosine, tryptophane et/ou aromatiques analogues.

12. Utilisation selon la revendication 11, **caractérisée en ce que** les composés contiennent jusqu'à quatre radicaux tyrosine, tryptophane et/ou aromatiques analogues.

13. Utilisation selon l'une des revendications 8 à 12, **caractérisée en ce que** les peptides sont choisis de telle manière qu'ils n'ont pratiquement pas d'effet physiologique spécifique à leur structure.

14. Utilisation selon l'une des revendications 8 à 13, **caractérisée en ce que** les peptides sont choisis parmi les hormones qui libèrent de la gonadotrophine, la dermorphine ainsi que leurs fragments tronqués N- ou C-terminaux.

15. Utilisation selon l'une des revendications 8 à 13, **caractérisée en ce que** les peptides sont choisis parmi le groupe consistant en : WY, WGY, WGGY, WGGGY, WW et YY.

16. Utilisation selon l'une des revendications précédentes, **caractérisée en ce que**, à l'endroit cible, les composés sont administrés de telle manière que la concentration en matière active est de 1 nM à 100 µM.

17. Utilisation selon l'une des revendications précédentes, **caractérisée en ce que** l'administration est systémique, en particulier par voie orale.

18. Utilisation selon l'une des revendications précédentes, **caractérisée en ce que** l'administration est locale, en particulier par application topique.

19. Formule de crème solaire **caractérisée en ce que**, outre les composants habituels, elle comprend une partie de peptides à effet antioxydant qui contiennent (a) au moins un radical tryptophane et au moins un radical tyrosine ou au moins deux radicaux tryptophane et/ou des radicaux aromatiques analogues et/ou (b) au moins deux radicaux tyrosine et/ou des radicaux aromatiques analogues et ont une masse moléculaire jusqu'à 2 500 Da.

20. Formule selon la revendication 19, **caractérisée en ce que** les peptides sont présents dans une proportion de 0,5 à 10 % en poids si on se réfère au poids total de la formule.
